# EUROPEAN PATENT SPECIFICATION

(11) **EP 3 563 900 B1**
(45) Date of publication and mention of the grant of the patent: **21.06.2023**
(21) Application number: 17888505.9
(22) Date of filing: 28.12.2017
(51) Int. Cl.: A61M 37/00, A61K 9/70, A61K 47/32, A61K 47/34, A61L 31/04, A61L 31/06, A61L 31/12, A61L 31/16, A61K 9/00

(54) **MICRONEEDLE ARRAY COATED WITH DRUG**
MIT EINEM ARZNEIMITTEL BESCHICHTETE MIKRONADELANORDNUNG
RÉSEAU DE MICRO-AIGUILLES REVÊTU DE MÉDICAMENT

(30) Priority: 28.12.2016 JP 2016256925
(43) Date of publication of application: 06.11.2019
(73) Proprietor: Cosmed Pharmaceutical Co., Ltd., Kyoto-shi, Kyoto 601-8014 (JP)
(72) Inventor: QUAN, Ying-shu, Kyoto-shi Kyoto 601-8014 (JP); SAITO, Mio, Kyoto-shi Kyoto 601-8014 (JP); YAMASHITA, Hirofumi, Kyoto-shi Kyoto 601-8014 (JP); KAMIYAMA, Fumio, Kyoto-shi Kyoto 601-8014 (JP)
(74) Representative: Ter Meer Steinmeister & Partner
(86) International application number: PCT/JP2017/047302
(87) International publication number: WO 2018/124290

(56) References cited:
- WO-A1-2016/084701
- WO-A1-2017/135290
- JP-A- 2014 079 557
- JP-A- 2014 507 473
- JP-A- 2015 109 963

## Description

### Technical Field

The present invention relates to a drug-coated microneedle array, and to the field of transdermal absorption preparations.

### Background Art

As a method of administering a drug to a human body, oral administration and transdermal administration are often used. Injection is a typical transdermal administration method. However, injection is a procedure which takes time and labor of specialists such as physicians and nurses and is painful so that many people do not welcome the procedure. In contrast, a transdermal administration method without pain using a microneedle array has been recently attracting attention (Non-patent Document 1).

In the transdermal administration of a drug, skin stratum corneum works as a barrier to drug permeation, so that enough permeability is not always provided by only applying the drug on a skin surface. In contrast, perforation of corneum by using a minute needle, i.e. a microneedle can remarkably improve drug permeation efficiency compared to the application method. An article in which a large number of the microneedles are integrated on a substrate is a microneedle array. In addition, a product in which an adhesive sheet for adhering the microneedle array to a skin, a release sheet for protecting an adhesive surface, and the like are added to the microneedle array to facilitate its use is called a microneedle patch.

An attempt to hold or apply a drug on a microneedle has been made. However, some drugs to be contained in the microneedle array are extremely expensive or can be obtained only in minute amounts. When such an expensive and valuable drug is contained in a base material to make the microneedle array, the drug would be contained not only in a microneedle portion but also in a substrate portion. When this microneedle array is inserted into a skin, the drug contained in the microneedle portion is incorporated and diffused in a body, but the drug remaining in the substrate portion is discarded without utilization, resulting in low usage efficiency of the expensive drug.

Some trials for efficient utilization of the expensive drug are already known. A method in which surfaces of the microneedles are coated with a drug by using a drug solution (Patent Documents 1 to 4) and a method in which a granulated drug is converged to tips of microneedles by centrifugation while the microneedles maintain softness (Patent Document 5) have been reported. The method of coating the surfaces of microneedles with a drug and the method of adhering a drug to the tips of microneedles from a drug solution have a problem that the drug must be heated or that the adhered drug falls away during insertion of the microneedles. In contrast, a method in which an adhered drug and a microneedle body were integrated by dissolving the drug in a solvent of the microneedle material to prevent the adhered drug from falling away was proposed (Patent Document 6).

The method of soaking the tips of microneedles into a drug solution to adhere the drug to the tips of microneedles is easily put into practical use due to its convenience (Patent Documents 1 to 4, 6). However, it is very difficult to quantitatively apply the drug to the tips of microneedles with little unevenness.

In case of a microneedle made of a hydrophobic material, the application of a drug by means of its aqueous solution is difficult, and thus quantitative loading with the drug is impossible. When a microneedle made of a hydrophilic material is only soaked into a drug aqueous solution, the drug aqueous solution is moved up from a bottom of the needle to a substrate portion by capillary phenomenon to widely distribute the drug so that it is impossible to quantitatively load the tip portion of the microneedle with the drug. Several hundred microneedles stand closely with intervals of 20 µm to 1,000 µm in one microneedle array, so the drug aqueous solution is extremely easily moved up by capillary phenomenon. Thus, although several trials have been repeated, it is extremely difficult that the microneedle array is soaked into the drug aqueous solution to a fixed depth to quantitatively hold the drug.

In order to prevent the capillary phenomenon, a method of masking any place other than the tip portion of microneedles before applying a drug has been proposed (Patent Document 2). A method of feeding a drug into a large number of holes with a spatula and then inserting the microneedles into the holes to enhance the quantitativity of a drug adhesion amount (Patent Document 4) has been also proposed. However, since execution of these methods is very complicated and furthermore technique for preventing the drug from falling away during insertion of the microneedles into a skin is not shown, the methods are considered to be insufficient for the quantitative administration of the drug.

In recent years, a microneedle having a step, wherein the step carries a drug, a tip of the microneedle is exposed, and the sharp tip portion is maintained (Patent Document 7), a technique in which a drug is held on a tip portion and a step, and thereby the drug is quantitatively administered (Patent Document 8) have also been developed.

### Prior Art Documents

### Patent Documents

[Patent Document 1] JP-2008029710A
[Patent Document 2] JP-2007521090A
[Patent Document 3] JP-2008520370A
[Patent Document 4] JP-2008139648A1
[Patent Document 5] JP-2009507573A
[Patent Document 6] JP-2011224308A
[Patent Document 7] JP-2015-109963A
[Patent Document 8] JP-2014-079557A

### Non-Patent Documents

[Non-Patent Document 1] YonSuk KA, Fumio KAMIYAMA "The Course of Productization of Microneedle", The Academy of Pharmaceutical Science and Technology, Japan; July 2009, Vol. 69, 4th issue, pp.272-276

### Summary of Invention

### Problem to be solved

The present inventors actually applied a microneedle array having a drug-coated microneedle tip portions to an animal, and subjected a skin corneum of the application site to tape stripping for measurement, and as a result, they found that part of the drug remained in a skin stratum corneum. Thus, as a problem, there was a possibility that, even if the tip portions of the microneedles could be quantitatively coated with the drug, the drug remained in the stratum corneum when actually applying the microneedle array as a transdermal absorption preparation, and the purpose of quantitatively administering the drug could not be achieved. An object of the present invention is to provide a practical microneedle preparation by reducing the drug remaining in the stratum corneum.

### Solution to Problem

In order to solve the above problem, the inventors of the present invention intensively examined the drug remaining in the stratum corneum for various microneedles having drug-coated tip portions, and as a result, found that the intended purpose could be achieved by combining a specific shape of microneedle with a specific ratio of drug application portion, and thus completed the present invention.

The present invention is as follows.
[1] A microneedle array having needles with a length of 350 µm to 900 µm, including drug-coated portions, and using a non-biosoluble polymer as a base, wherein a lower end of each drug-coated portion is 200 µm or more away from a root of the needle, and wherein the needles have a tip apex diameter of 30 µm to 60 µm.
[2] The microneedle array according to [1], wherein the non-biosoluble polymer is selected from a group consisting of nylon, polycarbonate, polylactic acid, poly(lactic acid-glycolic acid) copolymer, polyglycolic acid, polyethylene terephthalate, COP (cyclic olefin polymer) and a mixture thereof.
[3] The microneedle array according to [1], wherein the drug-coated portions concomitantly contain a water-soluble substance selected from a group consisting of hyaluronic acid, collagen, dextrin, dextran, sodium chondroitin sulfate, hydroxypropylcellulose, ethylcellulose, carboxymethylcellulose sodium salt, alginic acid, glucose, sucrose, maltose, trehalose, and a mixture thereof.

The microneedles formed on the microneedle array according to the present invention have a shape with a needle length of 350 µm to 900 µm, and a tip apex diameter of 30 µm to 60 µm.

The base of the microneedle array according to the present invention is a non-biosoluble polymer. Herein, the non-biosoluble polymer refers to a polymer having a property not to completely dissolve for at least 15 minutes after molded into microneedles and pierced into a skin. In the present invention, a polymer which can be easily injection-molded or press-molded is preferable, and is exemplified by a polymer selected from a group consisting of nylon, polycarbonate, polylactic acid, poly(lactic acid-glycolic acid) copolymer, polyglycolic acid, polyethylene terephthalate, COP (cyclic olefin polymer), and a mixture thereof.

Alternatively, the base of the microneedle array according to the present invention may be a polymer selected from a group consisting of hyaluronic acid, dextran, polyvinylpyrrolidone, sodium chondroitin sulfate, hydroxypropylcellulose, polyvinyl alcohol, and a mixture thereof as long as the polymer has a property not to completely dissolve for at least 15 minutes after molded into microneedles and pierced into a skin.

The microneedle array according to the present invention is characterized in that the lower end of the drug-coated portion is 200 µm or more away from the root of the needle. As long as the lower end of the drug-coated portion is 200 µm or more away from the root of the microneedle, the upper end may be at any level depending on the amount of the applied drug. Preferably, the upper end is at the tip of the microneedle.

### Effects of Invention

Among transdermal absorption preparations, for the microneedle array having the drug-coated needle tip portion, a drug permeation efficiency is significantly improved by piercing a skin stratum corneum acting as a barrier to drug permeation during transdermal administration of the drug. However, unexpectedly, it was found that a part of the drug remained in the skin stratum corneum. For the microneedle array according to the present invention, it was succeeded to specialize the shape of the microneedles and the position of the drug-coated portion to reduce the amount of the drug remaining in the stratum corneum. The microneedle array according to the present invention is a transdermal absorption preparation with an improved drug transdermal delivery efficiency. The microneedle array according to the present invention can deliver a trace amount of drug to a dermic layer with less burden on a body and transfer the drug from subcutaneous tissues to blood to maintain a blood drug level.

### Brief Description of Drawings

Figure 1 illustrates a schematic diagram of a microneedle having a step.
Figure 2 illustrates a photomicrograph showing an overview image of a microneedle array used in Examples.

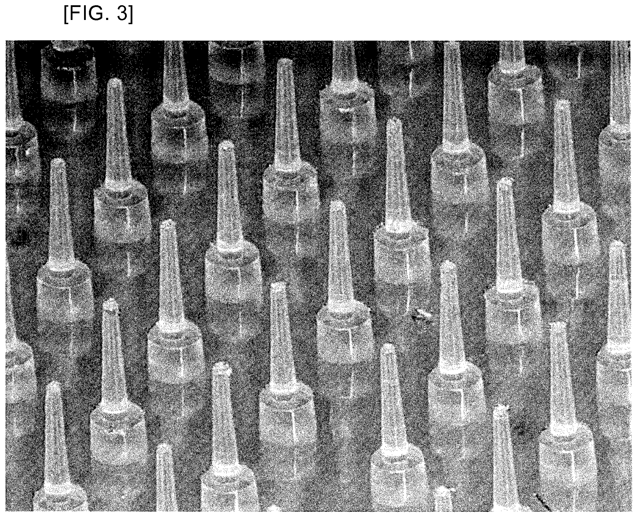

Figure 3 illustrates a photomicrograph showing a shape of a 400 µm-long needle of the microneedle array used in Examples.

### Description of Embodiments

### Shape of Microneedle

Microneedles constituting a microneedle array have a needle length of 300 µm to 900 µm, preferably 400 µm to 800 µm, more preferably 400 µm to 600 µm for ensuring transdermal absorption of a drug.

The needles have a tip apex diameter of 30 µm to 60 µm for facilitating piercing of the needles into a skin and reducing the drug remaining in the skin.

Each microneedle is in a cylindrical or conical shape with a circle bottom face, or in an elliptical cylindrical or elliptical conical shape with an elliptical bottom face. When expressing the size of the ellipse, the major axis is expressed as a diameter, and the minor axis is shorter than the major axis as long as an ellipse can be formed. The microneedles having these shapes may have steps.

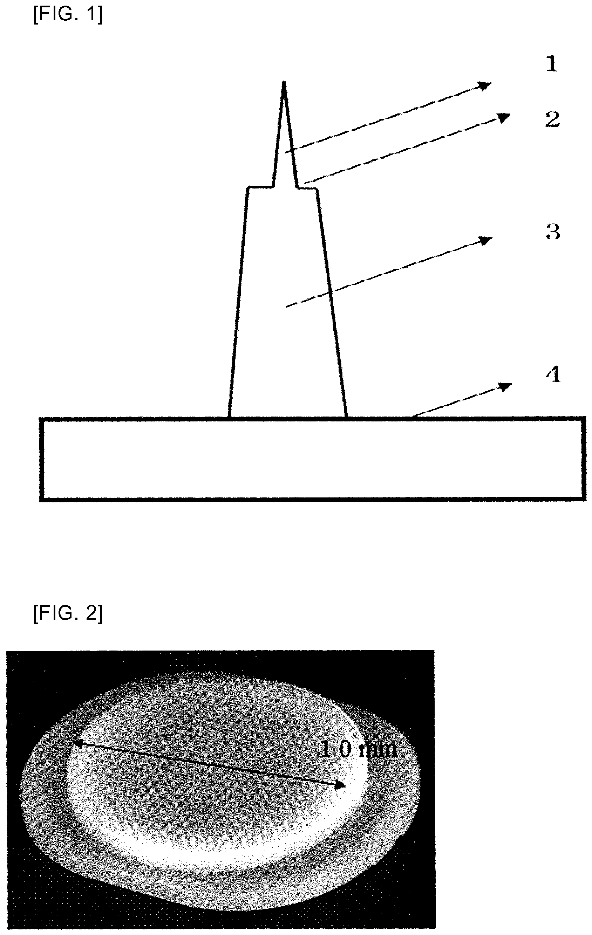

"Step" as used herein means a portion where a cross section area of the microneedle is reduced discontinuously from a certain point on the microneedle toward the tip and where a cross section presents a step-wise shape as shown in Figure 1. The shape of the microneedle having the step will be described with reference to Figure 1. In the stepped microneedle, preferably a length of a tip portion 1 is 50 µm to 500 µm, and the other portion is defined as a bottom portion 3. More preferably, out of the needle length of 300 µm to 900 µm, a length of the tip portion is 50 µm to 450 µm, and the other portion is defined as the bottom portion. A size of a margin 2 of the step formed by the tip portion and the bottom portion is preferably larger than 10 µm and smaller than 100 µm. More preferably, the size is 14 µm to 50 µm. A substrate of the microneedle array is numbered 4.

The margin 2 of the step is a face perpendicular to the axis of the microneedle (face parallel to the substrate) within a range of machining accuracy. In addition, the size of the margin 2 of the step refers to a difference in radius between the tip portion and the bottom portion on the step. The tip portion may be in a conical shape, a cylindrical shape, an elliptical cylindrical shape, or an elliptical conical shape depending on the shape of the microneedle. Similarly, the bottom portion may be in a conical shape, a cylindrical shape, an elliptical cylindrical shape, or an elliptical conical shape.

### Microneedle Having Drug-coated Portion

The microneedle array according to the present invention has a drug-coated portion. The drug-coated portion is at the tip portion of the microneedle, and when the tip of the microneedle is directed upward, the lower end of the drug-coated portion is 200 µm or more away from the root of the needle. When the lower end of the drug-coated portion is 200 µm or more away from the root of the needle, the upper end may be at any level depending on the amount of the applied drug. Although the upper end is preferably the tip of the microneedle, the end of the tip is not necessarily coated. A length of the drug-coated portion is typically 100 µm to 800 µm, preferably 150 µm to 600 µm.

The lower end and the upper end of the drug-coated portion are values obtained by respectively measuring the lower end and the upper end of the drug-coated microneedle in the vertical direction from the substrate of the microneedle array. The length of the drug-coated portion is represented by a difference between the lower end and the upper end of the drug-coated microneedle.

On the other hand, the thickness of the drug-coated portion varies depending on the liquid for drug application and the number of applications. In the present invention, the thickness of the coated portion can be represented by a diameter of a portion near an intermediate position of the drug-coated portion. The diameter of the portion near the intermediate position of the drug-coated portion is larger than the diameter of the microneedle before drug application. The present inventors have found that when not only the diameter of the drug-coated portion of the microneedle but also the diameter of the thickest part near an intermediate position of the drug-coated portion are in predetermined ranges, the drug remaining in the keratin is reduced, and a practical microneedle array can be achieved.

### Drug Applied to Microneedle

The drug is not particularly limited as long as it is a drug or a cosmetic raw material conventionally used as a transdermal absorption preparation. Examples of the drug include antipyretic analgesic, steroidal antiinflammatory agent, vasodilator, antiarrhythmic agent, antihypertensive agent, local anesthetic, hormone, antihistamine, general anesthetic, sedative hypnotic agent, anti-temper tantrum agent, psychoneurosis agent, skeletal muscle relaxant, autonomic agent, antiparkinsonian, diuretic, vasoconstrictor, respiratory stimulant, narcotic, antigenic components against pathogens (e.g. vaccine antigenic protein), and the like. A content of the medicinal ingredient can be appropriately set depending on a characteristic of the ingredient, a purpose of administration, an administration subject, a number of administrations, and the like.

Most of the drugs are low molecular weight compounds with a molecular weight of 600 or less, while some drugs with a high molecular weight can be used. Preferable drugs with a high molecular weight include, for example, bioactive peptides and derivatives thereof, nucleic acids, oligonucleotides, various antigen proteins, bacteria, virus fragments, and the like.

The above-mentioned bioactive peptides and the derivatives thereof include, for example, calcitonin, adrenocorticotropic hormone, epidermal growth factor (EGF), parathyroid hormone (PTH), hPTH (1→34), insulin, atrial natriuretic peptide, growth hormone, growth hormone-releasing hormone, endothelin, salts thereof, and the like. These medicinal ingredients can be administered not only to humans, but also to animals. The antigen proteins include influenza virus antigen, Japanese encephalitis virus antigen, diphtheria, tetanus antigens, HBs surface antigen, HBe antigen, and the like.

### Method for Producing Microneedle Array

The base of the microneedle is preferably a polymer which can be easily injection-molded or press-molded, and examples thereof include nylon, polycarbonate, polylactic acid, poly(lactic acid-glycolic acid) copolymer, polyglycolic acid, polyethylene terephthalate, COP (cyclic olefin polymer) and a mixture thereof. Alternatively, the non-biosoluble polymer may be hyaluronic acid, dextran, polyvinylpyrrolidone, sodium chondroitin sulfate, hydroxypropylcellulose, polyvinyl alcohol, and a mixture thereof as long as the non-biosoluble polymer has a property not to completely dissolve for at least 15 minutes after molded into microneedles and pierced into a skin.

The microneedle array can be mass-produced using a mold (die). A microneedle array composed of an injection-moldable polymer as a base may be produced by injection-molding a base using a die (e.g. method described in [0017] and [0018] in Japanese Patent Application Laid-Open No. 2003-238347). For the injection molding die, stainless steel, heat resistant steel, superalloy, or the like can be used. A typical die has 100 to 1000 cut portions corresponding to microneedles per square centimeter to form microneedle shapes. For the purpose of forming the cut portions, a fine processing means such as a grinder can be used.

When hyaluronic acid, dextran, polyvinylpyrrolidone, sodium chondroitin sulfate, hydroxypropylcellulose, polyvinyl alcohol, or a mixture thereof is used as a base, a base aqueous solution may be poured into a mold, dried, and then removed (e.g. method described in [0029] and [0031] in Japanese Patent Application Laid-Open No. 2009-273872).

### Drug Application

The drug is applied to the microneedle array by immersing the tips of the microneedles in a drug solution to hold the drug on the tips of the microneedles. The drug solution is typically an aqueous solution but may contain a solvent other than water for dissolving the drug. In addition, the drug may be either completely dissolved or dispersed in a solvent.

A coexistent substance is dissolved in the drug solution in advance, and it is desirable that the drug is held on the microneedles with the coexistent substance in drying after application. The coexistent substance should be a substance which does not lose stability of the drug, and, for example, the water-soluble polymer substance such as hyaluronic acid, collagen, dextrin, dextran, sodium chondroitin sulfate, hydroxypropyl cellulose, ethyl cellulose, carboxymethyl cellulose sodium salt, and alginic acid; the low molecular weight saccharides such as glucose, sucrose, maltose, and trehalose; or mixtures thereof are suitable.

If the coexistent substance is only the water-soluble polymer substance, a dissolution time of a coating in a skin at transdermal administration of the microneedles may be long. In contrast, a coating made of only the low molecular weight saccharides has insufficient mechanical strength. Thus, as the coexistent substance for the drug aqueous solution into which the microneedles are soaked, the mixture of the water-soluble polymer and the low molecular weight saccharides is desirable. In such case, a ratio of the low molecular weight saccharides to a total weight of the coexistent substance is desirably 80 % by weight or less.

A concentration of the coexistent substance in the drug solution is desirably 2 % to 50 %. If the concentration is less than 2 %, viscosity of the drug solution is low, so a coating adhesion amount in soaking is small. In contrast, if the concentration is more than 50 %, since the concentration in the drug solution is too high, drug application is not stable.

The immersion time and the number of immersions can be set depending on the affinity between the base of the microneedles and the drug solution, and the drug is applied so that the diameter of the portion near the intermediate position of the drug-coated portion is a desired value. After application, the microneedles are dried until the solvent is evaporated. The microneedles may be forcibly dried by natural drying, or by blowing dry air, nitrogen gas or the like. In the application, a drug solution reservoir is filled with the drug solution, into which the microneedle array is immersed from above to attach the drug to the needles. The device needs to be able to control the immersion depth with an accuracy of about 20 microns for precisely defining the immersion depth of the microneedle array.

### Transdermal Absorption Preparation

The transdermal absorption preparation according to the present invention is composed of a microneedle array having a drug-coated portion.

The transdermal absorption preparation according to the present invention is administered to an animal having skin tissues. Herein, examples of the animal include fishes, amphibians, reptiles, birds (e.g. poultries such as chicken, quail, turkey, duck, geese, call duck and ostrich), and mammals. Mammals may either include or not include humans. Examples of the mammals other than humans include rodents such as mouse, rat, hamster and guinea pig, pet animals such as rabbit, cat and dog, domestic animals such as pig, cow, horse, sheep and goat, and primates such as monkey, orangutan, gorilla and chimpanzee.

The transdermal absorption preparation according to the present invention is preferably administered to a mammal.

### Examples

Hereinafter, the present invention will be explained with reference to Examples, however, the present invention is not exactly limited to Examples.

### Example 1

Polyglycolic acid (Kuredux, kureha Co., Ltd.) as a raw material was injection-molded at an injection temperature of 260°C so as to manufacture microneedles with steps.

Needle tips of the microneedles made of polyglycolic acid (PGA) (hereinafter, referred to as PGA-MN) were coated with a solution containing FITC (fluorescein isothiocyanate) as a fluorescent dye, and subjected to a rat application experiment. The FITC-coated PGA-MN was applied to a rat and removed, then the application site was subjected to tape stripping to take keratin, and the tape was subjected to solvent extraction to measure a fluorescence intensity.

In this Example, FITC-coated PGA-MNs different in (1) heights of the needles and (2) diameters of the coated needle portions were subjected to the experiment to examine an amount of the FITC remaining in the keratin.

The needle tips of the microneedles made of PGA (hereinafter, referred to as PGA-MN) were coated with the solution containing FITC as a fluorescent dye, and subjected to a rat application experiment. The FITC-coated PGA-MN was applied to a rat and removed, then the application site was subjected to tape stripping to take keratin, and the tape was subjected to solvent extraction to measure a fluorescence intensity.

### Experimental Method

### 1. Test material

The PGA-MN shown in Figure 1 was used for the rat application experiment. In Figure 1, the PGA-MN substrate is in an elliptical shape having a major axis of 1.4 mm, a minor axis of 1.2 mm, and a thickness of 1.0 mm, and has a stage having a thickness of 1.0 mm on the upper part. The needle has a two-stage structure. Table 1 shows dimensions of two types of PGA-MNs having different needle heights subjected to the experiment.

**[Table 1]**

| | Needle Dimension | | | | |
|---|---|---|---|---|---|
| | Bottom Portion Diameter (m m) | Bottom Portion Height (m m) | Tip Apex Diameter (m m) | Diameter of Bottom Portion of Tip Portion (m m) | Tip Portion Height (m m) |
| 400µm P G A-MN | 0.13 | 0.10 | 0.034 | 0.062 | 0.30 |
| 600µm P G A-MN | 0.12 | 0.30 | 0.032 | 0.060 | 0.30 |

The materials used for application are as follows. Hydroxypropylcellulose (HPC-L), manufactured by Nippon Soda Co., Ltd. Fluorescein-4-isothiocyanate, manufactured by Dojindo Molecular Technologies, Inc.

### 2. Method for applying PGA-MN preparation

1) A coating liquid was prepared by mixing FITC dissolved in 0.1 mol/L of NaOH and 16% HPC-L aqueous solution.
2) The PGA-MN tips were immersed in the coating liquid contained in a container to attach the coating liquid to the needle tips.
3) The needles were dried in a desiccator overnight.

### 3. Application of PGA-MN preparation to rat

1) An abdomen of an 8-week-old male Wistar/ST rat was shaved with a shaver, limbs were fixed with the abdomen up, and the rat was anesthetized with somnopentyl.
2) The FITC-coated PGA-MN was pressed and pasted on the abdominal skin so as to stick the needles, and pressure-fixed by taping.
3) The PGA-MN-pasted rat was placed in a fixture and fixed so as not to move.
4) After 5 minutes or 1 hour, the PGA-MN was taken out.
5) After 10 minutes, the surface of the skin on which the PGA-MN had been applied was dried.

### 4. Tape stripping and extraction/measurement method

1) A circular tape with a diameter of 12 mm was pasted and pressed on the skin on which the FITC-coated PGA-MN had been applied, and then peeled off. This step was repeated 15 times to sample a keratin on the skin surface.
2) Fifteen pieces of tapes were divided into each 5 pieces, put into a 6 well plate, and subjected to extraction with 1 ml of 0.01 mol/L NaOH aqueous solution.
3) The FITC-coated PGA-MN of the same lot before application was similarly subjected to the extraction.
4) The extract was put into a 96-well plate to measure fluorescence intensities at an excitation wavelength of 485 nm and at a measurement wavelength of 535 nm. As the measuring apparatus, a plate reader SpectraFluor manufactured by Tecan Japan Co., Ltd. was used.
5) A ratio of a fluorescence intensity of each extract of 15 tapes removed with the keratin relative to 100 of the fluorescence intensity of the MN before application was calculated, which was defined as an amount of the drug remaining in the keratin.

The experimental results are summarized as follows.

**[Table 2]**

| Summary of Rat-Application Experiment FITC-coated PGA-MN | | | | | |
|---|---|---|---|---|---|
| Condition | Needle Height (µm) | Application amount of FITC (ng) | Length of Coated Portion (µm) | Diameter of Coated Portion (µm) | Amount of Drug Remaining in Keratin (%) |
| Removed after 5 Minutes | 400 | 110 | 210 | 72 | 12.2 |
| | | 200 | 210 | 89 | 44.4 |
| | 600 | 80 | 190 | 63 | 1.2 |
| | | 140 | 190 | 78 | 7.4 |
| Removed after 1 Hour | 400 | 110 | 210 | 74 | 19.1 |
| | | 200 | 210 | 85 | 30.2 |
| | 600 | 80 | 190 | 68 | 11.2 |
| | | 140 | 190 | 78 | 8.8 |

### 5. Discussion

The experimental results showed that the reason why the amount of the drug remaining in the keratin after application was small was that (1) the height of the needle was high, and (2) the MN had a small diameter of the coated portion. It is considered that when a diameter of a coated portion of a 600 µm-long PGA-MN is 70 µm or smaller, an amount of the drug remaining in the keratin is about 10% or less, so that a practical microneedle preparation can be obtained.

### Reference Numerals

- 1: tip portion
- 2: margin of step
- 3: bottom portion
- 4: substrate of microneedle array

## Claims

1. A microneedle array having needles with a length of 350 µm to 900 µm, including drug-coated portions, and using a non-biosoluble polymer as a base, wherein a lower end of each drug-coated portion is 200 µm or more away from a root of the needle,
**characterized in that** the needles have a tip apex diameter of 30 µm to 60 µm.

2. The microneedle array according to claim 1, wherein the non-biosoluble polymer is selected from a group consisting of nylon, polycarbonate, polylactic acid, poly(lactic acid-glycolic acid) copolymer, polyglycolic acid, polyethylene terephthalate, COP (cyclic olefin polymer) and a mixture thereof.

3. The microneedle array according to claim 1, wherein the drug-coated portions concomitantly contain a water-soluble substance selected from a group consisting of hyaluronic acid, collagen, dextrin, dextran, sodium chondroitin sulfate, hydroxypropylcellulose, ethylcellulose, carboxymethylcellulose sodium salt, alginic acid, glucose, sucrose, maltose, trehalose, and a mixture thereof.

## Patentansprüche

1. Mikronadelanordnung mit Nadeln mit einer Länge von 350 µm bis 900 µm, einschließlich arzneimittelbeschichteter Abschnitte, und unter Verwendung eines nicht biolöslichen Polymers als Basis, wobei ein unteres Ende jedes arzneimittelbeschichteten Abschnitts 200 µm oder mehr von einer Wurzel der Nadel entfernt ist,
**dadurch gekennzeichnet, dass** die Nadeln einen Spitzendurchmesser von 30 µm bis 60 µm aufweisen.

2. Mikronadelanordnung nach Anspruch 1, wobei das nicht biolösliche Polymer aus einer Gruppe ausgewählt ist, die aus Nylon, Polycarbonat, Polymilchsäure, Poly(milchsäure-glykolsäure)-Copolymer, Polyglykolsäure, Polyethylenterephthalat, COP (cyclisches Olefinpolymer) und einer Mischung davon besteht.

3. Mikronadelanordnung nach Anspruch 1, wobei die arzneimittelbeschichteten Abschnitte gleichzeitig eine wasserlösliche Substanz enthalten, die aus einer Gruppe ausgewählt ist, die aus Hyaluronsäure, Kollagen, Dextrin, Dextran, Natriumchondroitinsulfat, Hydroxypropylcellulose, Ethylcellulose, Carboxymethylcellulose-Natriumsalz, Alginsäure, Glucose, Saccharose, Maltose, Trehalose und einer Mischung davon besteht.

## Revendications

1. Matrice de microaiguilles ayant des aiguilles d'une longueur de 350 µm à 900 µm, incluant des parties revêtues de médicament, et utilisant un polymère non biosoluble comme base, dans laquelle une extrémité inférieure de chaque partie revêtue de médicament est à 200 µm ou plus d'une base de l'aiguille,
**caractérisée en ce que** les aiguilles ont un diamètre de sommet d'embout de 30 µm à 60 µm.

2. Matrice de microaiguilles selon la revendication 1, dans laquelle le polymère non biosoluble est choisi parmi un groupe constitué de nylon, de polycarbonate, d'acide polylactique, d'un copolymère d'acides poly(lactique-glycolique), d'acide polyglycolique, de polyéthylène téréphtalate, de COP (polymère de cyclo-oléfine) et d'un mélange de ceux-ci.

3. Matrice de microaiguilles selon la revendication 1, dans laquelle les parties revêtues de médicament contiennent de manière concomitante une substance soluble dans l'eau choisie parmi un groupe constitué d'acide hyaluronique, de collagène, de dextrine, de dextrane, de sulfate de chondroïtine sodique, d'hydroxypropylcellulose, d'éthylcellulose, de sel de carboxyméthylcellulose sodique, d'acide alginique, de glucose, de saccharose, de maltose, de tréhalose et d'un mélange de ceux-ci.
